# DEMANDE DE BREVET EUROPEEN

(11) **EP 0 955 545 A1**
(43) Date de publication de la demande: **10.11.1999**
(21) Numéro de dépôt: 99401025.4
(22) Date de dépôt: 27.04.1999
(51) Int. Cl.: G01N 33/569, A61K 39/02

(54) **Procédé de sélection de souches bactériennes**

(30) Priorité: 30.04.1998 FR 9805559
(71) Demandeur: SANOFI SANTE NUTRITION ANIMALE, 33501 Libourne (FR)
(72) Inventeur: Butty, Pascal Jean-Luc, 34090 Montpellier (FR); Jeyasingham, Marina, Aberdeen AB11 6JD (GB)
(74) Mandataire: Le Guen, Gérard

(57) **Abrégé**

Cette invention concerne un procédé de sélection de souches bactériennes non pathogènes capables de se fixer sur les sites récepteurs d'adhésion tissulaire de souches bactériennes pathogènes, comprenant les étapes consistant à :
a) mettre en contact *in vitro* lesdits récepteurs avec une souche bactérienne à tester ;
b) ajouter un anticorps dirigé contre lesdits récepteurs, ledit anticorps étant éventuellement marqué de manière détectable ;
c) dans le cas où ledit anticorps n'est pas lui-même marqué de manière détectable, ajouter un agent de détection de l'anticorps ;
d) détecter la présence du complexe formé par ledit anticorps et lesdits récepteurs.

## Description

La présente invention a trait à un procédé de sélection de souches bactériennes non pathogènes utiles pour prévenir ou traiter les infections médiées par l'adhésion tissulaire de souches bactériennes pathogènes.

Dans de nombreuses infections bactériennes, on peut observer une phase précoce au cours de laquelle l'agent pathogène adhère à des sites particuliers de l'organisme hôte.

Cette adhésion permet en particulier aux bactéries pathogènes de s'installer dans des sites d'infection, tels que les muqueuses, qui sont continuellement lavés par leurs propres sécrétions et qui peuvent être animés de mouvements comme le péristaltisme. L'adhésion peut aussi aider une bactérie pathogène dans sa compétition avec la microflore de l'hôte.

L'adhésion d'une bactérie pathogène sur un tissu est fréquemment stéréospécifique et ne peut avoir lieu que si le tissu porte un type de récepteur bien particulier. L'interaction entre une lectine bactérienne et un sucre tissulaire est un exemple typique d'interaction stéréospécifique. Ces molécules de lectines sont souvent portées par des appendices filamenteux appelés fimbriae.

Les fimbriae sont de très fins filaments protéiques trouvés principalement, et très communément, chez les bactéries Gram-négatives. Elles peuvent se répartir sur toute la surface de la cellule ou être plus localisées. Une fimbriae consiste en sous-unités protéiques linéaires répétées. Ces sous-unités sont souvent riches en acides aminés non polaires, si bien que les cellules porteuses de fimbriae tendent à avoir des surfaces plus hydrophobes que celles des cellules qui en sont dépourvues. Chez les bactéries Gram-négatives, de nombreux types de fimbriae assurent l'adhésion des cellules entre elles ou des cellules à une surface quelconque. Chaque type de fimbriae possède en effet sa lectine et n'adhère qu'à un récepteur spécifique composé d'épitopes glyco-protéiques.

L'adhésion par des types de fimbriae spécifiques est en particulier essentielle à la virulence des souches entérotoxinogènes d'*Escherichia coli* (souches ECET). Ces souches adhèrent à la muqueuse duodénale et produisent des entérotoxines responsables, par exemple, de diarrhées.

Les auteurs de la présente invention ont à présent mis au point une méthode pour prévenir la fixation tissulaire de bactéries pathogènes, ladite méthode mettant en oeuvre des souches bactériennes non pathogènes sélectionnées pour leur capacité à se fixer sur les sites récepteurs des bactéries pathogènes avec une affinité supérieure ou égale à celles-ci.

La présente invention a plus particulièrement pour objet un procédé de sélection de souches bactériennes non pathogènes capables de se fixer sur les sites récepteurs d'adhésion tissulaire de souches bactériennes pathogènes, comprenant les étapes consistant à :
a) mettre en contact *in vitro* lesdits récepteurs avec une souche bactérienne à tester ;
b) ajouter un anticorps dirigé contre lesdits récepteurs, ledit anticorps étant éventuellement marqué de manière détectable ;
c) dans le cas où ledit anticorps n'est pas lui-même marqué de manière détectable, ajouter un agent de détection de l'anticorps ;
d) détecter la présence du complexe formé entre ledit anticorps et lesdits récepteurs isolés.

La détection du complexe formé entre ledit anticorps et lesdits récepteurs peut donc être réalisée de manière directe, en marquant ledit anticorps de manière détectable, ou de manière indirecte, en utilisant un agent de détection de l'anticorps.

Par l'expression "anticorps marqué de manière détectable", on entend que l'anticorps est conjugué ou couplé à un groupe de marquage.

Le groupe de marquage pourra être de nature diverse comme, par exemple, radioisotopique (tel que I¹²⁵, H³), enzymatique (notamment au moyen de phosphatase alcaline, peroxydase du raifort ou β-galactosidase), fluorescent, (notamment au moyen de fluorescéine ou de rhodamine), particulaire, (notamment au moyen de latex ou d'or colloïdal). Ces groupes de marquage sont bien connus de l'homme du métier.

La conjugaison ou couplage des divers marqueurs sur l'anticorps est effectué par des méthodes classiques (couplage au glutaraldéhyde, carbodiimide, anhydride maléique, succinique, agents hétérobifonctionnels etc.).

"L'agent de détection de l'anticorps" désigne tout moyen permettant de détecter indirectement ledit anticorps. Ledit agent de détection peut être notamment un deuxième anticorps dirigé contre ledit anticorps à détecter (ou premier anticorps), ledit deuxième anticorps étant marqué de manière détectable.

Selon l'invention, diverses méthodes de détection peuvent être mises en oeuvre : test radio-immunologique (RIA), test immuno-enzymatique (EIA, ELISA), test fluoro-immunologique (FIA), test immunochimioluminescent (CLIA), test par immunoagglutination (IA), immunonéphélémétrie, etc.

La détection du complexe formé entre ledit premier anticorps et ledit récepteur peut être également réalisée par une méthode immunocyto-chimique de type Davidoff, qui combine la technique de double peroxydase- anti-peroxydase et celle du complexe avidine-biotine-peroxydase.

Selon un mode préférentiel de réalisation du procédé de l'invention. la souche bactérienne à tester est mise en contact avec lesdits récepteurs à une concentration décroissante en bactéries. L'objectif est de rechercher, parmi diverses souches bactériennes testées, celle capable d'exercer l'inhibition la plus marquée de la fixation de l'anticorps sur le récepteur, pour la dilution la plus élevée possible en bactéries en se référant au profil des témoins négatifs et positifs.

Les anticorps dirigés contre les récepteurs d'adhésion tissulaire des souches bactériennes pathogènes peuvent être des anticorps mono- ou polyclonaux ou leurs fragments, anticorps chimériques ou immunoconjugués.

Des anticorps polyclonaux peuvent être obtenus à partir du sérum d'un animal immunisé avec ledit récepteur purifié selon les modes opératoires usuels.

De manière préférentielle cependant, on utilise des anticorps monoclonaux qui peuvent être obtenus selon la méthode classique de culture d'hybridomes décrite par Köhler et Milstein, à partir de préparations de récepteurs d'adhésion tissulaire purifiés.

Les anticorps peuvent être des anticorps chimériques, des anticorps humanisés, des fragments Fab et F(ab')2. Ils peuvent également se présenter sous forme d'immunoconjugués ou d'anticorps marqués.

Parmi les souches bactériennes dont la pathogénicité est médiée par une adhésion tissulaire, on peut citer notamment les souches entérotoxinogènes d'*E*. *coli,* une des plus répandues étant la souche identifiée par son sérotype ECET/0147 : K88ac (Erickson et al., Infection and Immunity, 1992, 60:983-988).

Les bordures en brosse des entérocytes de porcelets sensibles expriment un récepteur qui permet la fixation massive de la souche de ce pathogène. La fixation de la bactérie est aussitôt suivie d'une cascade de réactions moléculaires intra-membranaires dans l'entérocyte qui bouleverse les flux ioniques entraînant une perte d'eau qui, généralisée à tout l'intestin, finit par déshydrater irréversiblement le porcelet jusqu'à la mort. Dans un autre exemple, les fimbriae de *E. Coli* K99 se fixent sur des céramides N-glycolylneuraminyllactosyles mais pas sur des dérivés N-acétylés de l'acide sialique. Les liaisons N-glycolyles sont surtout présentes chez les animaux et notamment les bovins qui sont très affectés par *E. coli* K99.

Parmi les souches bactériennes dont la pathogénécité est médiée par une adhésion tissulaire, on peut également citer les Salmonelles. Chez ces bactéries, les fimbriae sont indispensables pour initier une colonisation en particulier dans le caecum des volailles, étape préliminaire à l'infection. L'adhésion de pili est également indispensable pour que *Neisseria Gonorrhoeae* exerce sa virulence. En outre, les bactéries *Streptococcus mutans, S. sobrinus. S. cricetus* et *S*. *rattus,* qui sont les bactéries les plus fréquemment rencontrées dans les caries dentaires, sont également visées. Ces bactéries présentent la particularité de transformer le sucrose en glycanes par l'excrétion de glycosyltransférases. Ces enzymes sont très importantes pour l'adhésion car le glycane ainsi formé se fixe sur l'émail et la gencive avec des particules alimentaires et se trouve être un récepteur très efficace pour les lectines portées par ces Streptocoques. Une fois fixés, les Streptocoques se multiplient et provoquent des lésions dentaires. Dans le cas du pathogène *Campylobacter jejuni,* ce sont les lipopolysaccharides qui jouent la fonction d'adhésine permettant la fixation aux cellules épithéliales et au mucus provoquant un état diarrhéique sévère chez l'homme. *Clostridium difficile* doit également s'associer à la muqueuse intestinale pour être virulent. *Yersinia enterolytica* ainsi que *Pseudomonas aeruginosa* exercent leur pathogénécité après s'être fixés par l'intermédiaire de protéines localisées dans la membrane externe jouant le rôle d'adhésine. *Helicobacter pylori* est responsable de maladies inflammatoires gastriques chroniques et d'ulcérations gastriques et duodénales. L'adhésion *d'Helicobacter* sur les cellules gastriques par le biais des adhésines à la surface du pathogène se fixant à un récepteur sur la muqueuse gastrique est primordiale pour déclencher le processus de l'ulcération.

Le procédé de la présente invention est particulièrement avantageux pour sélectionner les souches bactériennes non pathogènes capables de se fixer sur des récepteurs spécifiques de la paroi intestinale sur lesquels se fixent les souches entérotoxinogènes *d'Escherischia coli.*

Une présélection des souches bactériennes à tester peut être avantageusement effectuée. Dans le cas de l'infection par des souches ECET, les souches à pré-sélectionner, qui peuvent être choisies notamment parmi les lactobacilles, sont testées pour leur capacité à reconnaître et à se fixer sur des fragments de duodénum et des entérocytes isolés de porcelets sensibles aux infections colibacillaires

Dans le cas des infections par des Salmonelles aviaires, des bactéries anaérobies sont avantageusement testées sur des fractions tissulaires d'entérocytes isolés du caecum. Dans le cas des *E*. *coli* aviaires, les lactobacilles seraient intéressants à tester sur des fractions tissulaires de cellules trachéales (cas des colibacilloses respiratoires) ou des entérocytes (cas des colibacilloses digestives). Enfin, les lactobacilles et les bifidobactéries sont des candidats intéressants dans le cas d'autres pathogènes isolés du tube digestif ou urinaire d'un organisme hôte humain ou animal infecté.

Les auteurs de la présente invention ont ainsi sélectionné les souches référencées D1, 27S et 30S, qui entrent en compétition avec les souches entérotoxinogènes d'*E*. *coli* pour la fixation aux fimbriae des récepteurs intestinaux tels que décrits précédemment et sont utiles pour s'opposer à l'expression de la pathogénicité desdites souches ECET. Les souches 27S et 30S ont été isolées du petit intestin de porcelets âgés de 6 à 16 semaines et ont été identifiées comme étant deux bactéries lactiques faisant partie du genre *Lactobacillus* et du groupe Il des lactobacilles hétérofermentaires stricts, espèce de *Lactobacillus fermentum.* La souche D1 est une bactérie lactique faisant partie du genre *Lactobacillus* et du groupe I des lactobacilles homofermentaires stricts, espèce de *Lactobacillus salivarius.*

Par "organisme hôte", on entend un humain ou un animal non humain, qui héberge les souches bactériennes visées. Parmi les animaux considérés comme des organismes hôtes, on peut citer notamment le porc, en particulier le porcelet, la vache, en particulier le veau, ainsi que le mouton, les volailles, en particulier le poulet, etc.

Le porcelet est particulièrement sensible aux toxines sécrétées par de nombreuses souches de colibacilles entérotoxinogènes, en particulier après le sevrage, pendant la période où il n'est plus protégé par les anticorps du lait maternel, avant que sa propre production d'anticorps ne soit établie.

La présente invention a également pour objet une méthode de traitement thérapeutique selon laquelle une quantité efficace des souches bactériennes non pathogènes sélectionnées par le procédé selon l'invention est administrée à un sujet, humain ou animal, nécessitant un tel traitement.

La présente invention a ainsi pour objet une composition thérapeutique comprenant une souche bactérienne sélectionnée par le procédé de l'invention, en association avec un véhicule pharmaceutiquement acceptable.

Les compositions pharmaceutiques selon l'invention peuvent être administrées notamment par voie orale.

Leurs modes d'administration, posologies et formes galéniques optimaux peuvent être déterminés selon les critères généralement pris en compte dans l'établissement d'un traitement thérapeutique adapté à un patient comme par exemple l'âge ou le poids corporel du patient, la gravité de son état général, la tolérance au traitement et les effets secondaires constatés, etc.

Les souches bactériennes sélectionnées par le procédé selon l'invention peuvent également être administrées aux animaux par incorporation dans la boisson ou les aliments, tels que notamment les aliments de gavage, les soupes ou tout autre aliment liquide ou pâteux.

La présente invention a également pour objet l'utilisation des souches bactériennes non pathogènes sélectionnées par le procédé tel que défini précédemment pour la fabrication d'une composition thérapeutique destinée à prévenir ou traiter les troubles pathologiques associés à une infection d'un organisme hôte par des souches bactériennes pathogènes se fixant sur lesdits récepteurs médiant l'adhésion tissulaire.

Parmi les troubles pathologiques visés, on peut citer notamment ceux associés à une infection par des souches entérotoxinogènes d'*Escherischia coli.*

Les exemples et la figure suivants illustrent l'invention sans la limiter d'une quelconque façon.

### LEGENDE DES FIGURES :

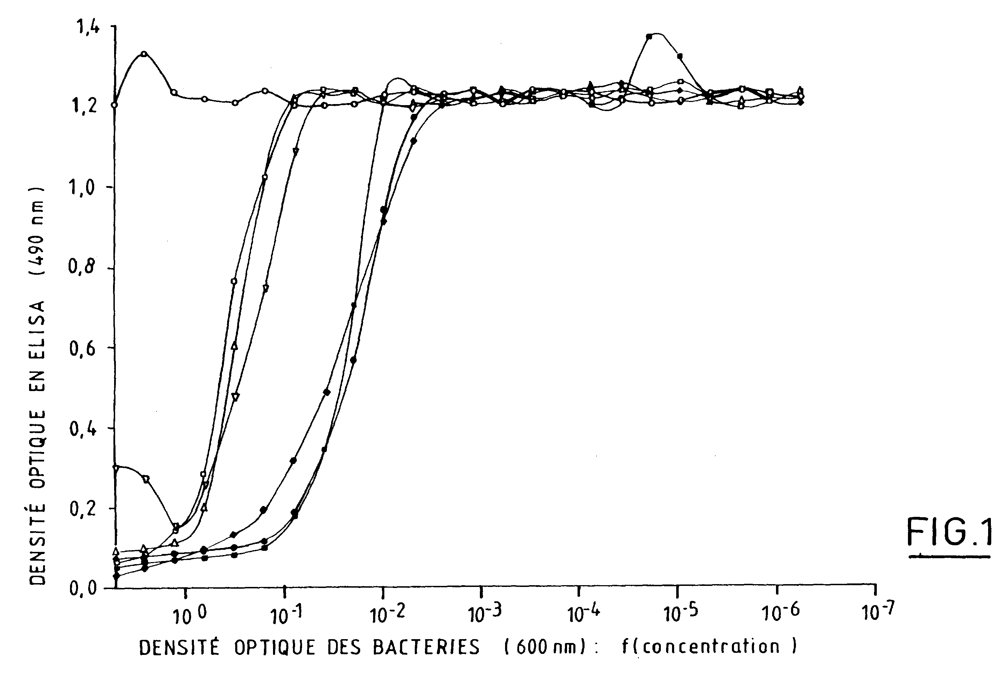

La figure 1 en annexe représente le résultat d'un test ELISA sur une préparation de récepteur intestinal à 10 µg/ml avec cinq souches bactériennes, en présence d'un anticorps IgM monoclonal anti-récepteur (référencé 7E8aB4) (0,24 µg/ml), et d'un anticorps anti-Igm marqué à la peroxydase.

Les souches testées sont la souche 27S (●), 30S (■), 36S (Δ), LB14 (∇) et D1 (◆). Un contrôle positif (○) a été réalisé en présence d'un anticorps 7E8aB4 et d'un anticorps anti-lgM marqué à la peroxydase, en l'absence de toute souche bactérienne. Un contrôle négatif (□) a été réalisé en présence d'un anticorps 7E8aB4 et d'un anticorps anti-Igm marqué à la peroxydase et de la souche 3A.

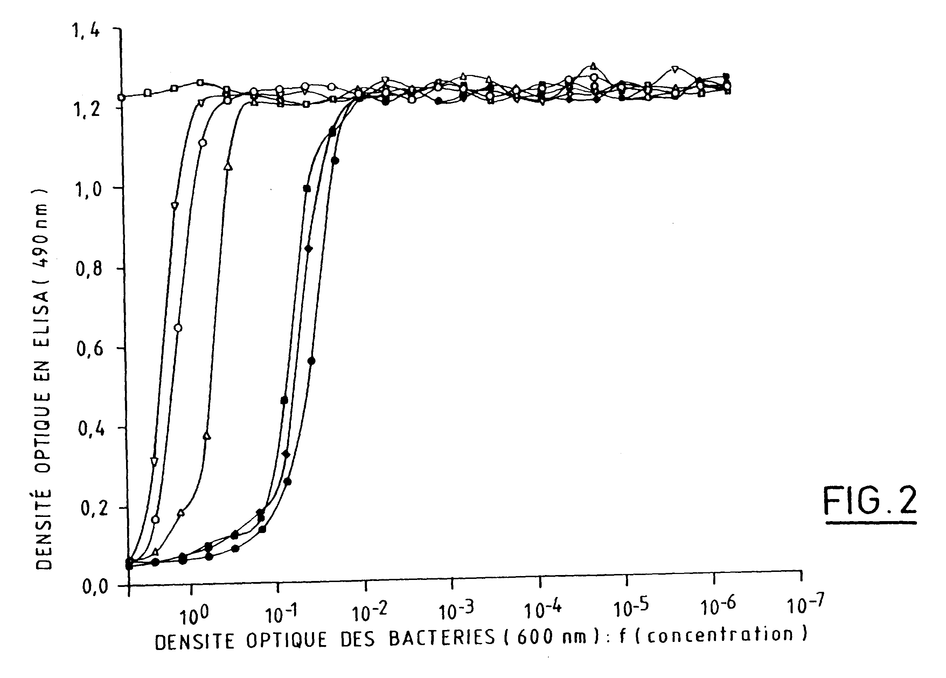

La figure 2 en annexe représente le résultat d'un test ELISA sur une préparation de récepteur intestinal à 10 µg/ml avec cinq souches bactériennes, en présence de fimbriae K88ac purifiées (12,5 µg/ml), de sérum anti-K88ac, IgG anti-sérum biotinylés et du complexe streptavidine-peroxydase de raifort.

Les souches testées sont la souche 27S (●), 30S (■), 36S (Δ). LB14 (∇) et D1 (◆). Un contrôle positif (○) a été réalisé en présence de fimbriae K88ac purifiées (12,5 µg/ml), de sérum anti-K88ac, IgG anti-sérum biotinylés et du complexe streptavidine-peroxydase de raifort, en l'absence de toute souche bactérienne. Un contrôle négatif (□) a été réalisé en présence de fimbriae K88ac purifiées (12,5 µg/ml), de sérum anti-K88ac, IgG anti-sérum biotinylés, du complexe streptavidine-peroxydase de raifort et de la souche 3A.

### EXEMPLE :

### Sélection de souches bactériennes non pathogènes capables de se fixer aux sites récepteurs d'un colibacille entérotoxinogène.

### I - la purification des récepteurs

De nombreux travaux ont été entrepris pour purifier le récepteur (Cohen et al., Fractionation and characterization of mouse small intestine mucus and brush border receptors for the K88ab adhesin, Microecology and Therapy, 1985, 15: 71-83 et Erickson et al., Infection and Immunity, 1992, 60 : 983-988). Toute la difficulté consiste à obtenir un rendement suffisant de récepteurs actifs pour lancer la production d'anticorps monoclonaux.

Des porcelets sensibles aux colibacilloses expérimentales, donc exprimant des récepteurs aux colibacilles ont été sélectionnés et séparés des animaux résistants dépourvus de récepteurs (Valpotic I et al., Veterinarski Arhiv. 1989, 59: 161-175 et Valpotic I. et al., Periodicum Biologorum, 1990, 92:43-44). La muqueuse de l'intestin grêle a été ensuite soigneusement prélevée et homogénéisée en présence d'inhibiteurs de protéases. Les vésicules de la bordure en brosse des entérocytes ont été séparées des composants du mucus, puis les récepteurs hautement glycosylés ont été extraits des vésicules par des traitements ultra-sons et par le jeu de précipitations alcooliques et phénoliques. La phase aqueuse enrichie en récepteurs a enfin été dialysée, puis lyophilisée. Après chaque étape de purification, le récepteur a été testé par western blot pour sa capacité à fixer les fimbriae de la bactérie K88ac.

Le protocole de purification détaillé est le suivant :

La purification du récepteur commence par la production des vésicules de bordures en brosse.

Dans cette première étape, la muqueuse intestinale est soigneusement prélevée d'un segment intestinal de 10 cm puis déposée dans un Potter. Un volume de tampon égal à cinq fois la masse de muqueuse est constitué de 50 mM de mannitol, 2 mM de Trisma-HCI de pH 7,4, et des inhibiteurs de protéases [PMSF (fluorure de phényl-méthyl-sulfonyle), inhibiteur de trypsine, Pepstatine A et Leupeptine] est ajouté dans le Potter. La suspension est alors broyée par 15 aller-retours du piston. Un volume du même tampon égal à cinq fois le volume de la suspension est ensuite ajouté. La suspension est de nouveau broyée par quinze aller-retours du piston. Le volume de la solution estimé entre 7,5 et 17,5 ml est alors mis en contact 20 minutes à 0°C avec une solution de MgCl₂ pour atteindre un pourcentage de 1 %. Deux centrifugations de 3000 et 27 000 g pendant respectivement quinze à trente minutes sont réalisées afin de récupérer le culot resolubilisé dans 100 µl de tampon. La concentration en protéines est ensuite déterminée.

Les récepteurs sont ensuite extraits des vésicules de bordures en brosse pour être semi-purifiés.

Dans cette seconde étape, du détergent CHAPS 3-[(3-cholamidopropyl)-diméthyl-ammonio]-1-propane sulfonate correspondant à cinq fois la quantité de protéines est ajouté aux 100 µl de suspension. Les protéines sont solubilisées dans un potter par 15 aller-retours du piston. La solubilisation se poursuit pendant douze heures à 4°C. La suspension est alors centrifugée à 125 000 g pendant une heure. Le surnageant est prélevé, dialysé pendant 24 heures à 4°C (MWCO : Molecular Weight Cut Off 12 000-14 000) et centrifugé pendant une heure à 125 000 g. Le surnageant est repris pour être de nouveau dialysé et centrifugé comme précédemment deux fois de suite. Après chaque centrifugation, le culot est resolubilisé dans de l'eau distillée. Le volume final de solution ne dépasse pas 1 ml.

Les récepteurs semi-purifiés sont ensuite traités pour l'obtention de récepteurs purifiés.

Dans cette troisième étape, la préparation est d'abord soumise à des ultrasons (15 watts) pendant trois fois 15 secondes dans de la glace fondante. Elle est ensuite centrifugée à 125 000 g pendant 40 minutes. Le culot est une nouvelle fois resolubilisé dans 1 ml d'eau distillée et le traitement aux ultrasons est de nouveau réalisé. Cette manipulation est renouvelée douze fois. Après chaque étape de centrifugation, le surnageant est déposé dans un tube en contact avec de la glace fondante. La totalité du surnageant est concentrée en tube de dialyse (MWCO : 10 000) contre du PEG solide jusqu'à obtention d'un tiers du volume initial. La préparation concentrée est alors diluée dans de l'éthanol en ajustant la préparation à 11 % d'alcool puis elle est incubée pendant vingt minutes dans de la glace fondante. La préparation est ensuite centrifugée à 125 000 g pendant quarante minutes. La pureté du culot en récepteurs est estimée par une analyse en électrophorèse SDS-PAGE. Les récepteurs sont traités successivement de la même manière que précédemment mais dans des solutions d'éthanol à 20, 33, 43, 55 et 64 %. Les récepteurs sont recueillis à partir des précipitations alcooliques à 43, 55 et 64 %. Ils sont solubilisés dans de l'eau distillée puis lyophilisés.

### II - la production d'anticorps monoclonaux spécifiques des récepteurs

La production d'anticorps monoclonaux spécifiques des récepteurs a été réalisée selon une méthode dérivée de Köhler et Milstein (Nature, 1975. vol. 256, pp 495-497).

Les récepteurs purifiés selon le procédé décrit ci-dessus ont été injectés à des souris albinos Balb/c. La recherche des meilleurs titres sériques en anticorps a permis de sélectionner trois souris choisies pour trois fusions cellulaires. La fusion a consisté à prélever, à partir de la rate de la souris, les lymphocytes B sécrétant des anticorps, et de faire fusionner ces cellules avec des cellules lymphoblastiques cancéreuses. La croissance sélective des hybridomes en culture a été assurée par le jeu d'inhibiteurs de synthèse de l'ADN et l'activité des anticorps anti-récepteur des surnageants de culture a été testée.

Les expériences suivantes ont consisté à isoler les cellules à raison d'une cellule hybride par puits. Chaque surnageant de chacun des puits contenant des cellules toutes identiques est testé en ELISA pour une recherche de la meilleur activité anti-récepteur. Les clones intéressants ont ensuite été injectés à des souris conditionnées au pristane pour la production massive d'anticorps en liquide d'ascite, lesquels ont enfin été purifiés et caractérisés (J. H. Peters et al., Monoclonal antibodies, 1992, 475 et L. Hudson et al., Practical Immunology, 1989 : 496).

### III - la validation des anticorps monoclonaux

A partir des clones obtenus, 20 anticorps ont été testés par trois techniques (immunocytochimie, ELISA et BlAcore) pour leur capacité à reconnaître le récepteur intestinal. Six anticorps ont été retenus. Ces anticorps ont été ensuite analysés en ELISA et BlAcore pour leur capacité à inhiber la fixation des fimbriae de K88ac sur leur récepteur par compétition moléculaire. Deux anticorps ont inhibé la fixation des adhésines bactériennes à 100 % et ont été choisis comme outils immunologiques dans le test de sélection ci-dessous. Un des anticorps monoclonaux produit est identifié par la référence 7E8aB4.

### IV - la sélection de souches bactériennes

Les souches testées ont été prélevées sur des porcs à l'abattoir. Ces souches appartiennent aux genres et espèces *Lactobacillus fermentum, L. delbrueckii, L. acidophilus, L. salivarius, Leuconostoc lactis,* cette liste n'étant pas exhaustive. Ces souches ont fait l'objet de tests préliminaires pour vérifier leur capacité d'adhésion à la muqueuse intestinale, et aux entérocytes isolés.

### a) Description du protocole Composition du milieu MRS :

| | |
|---|---|
| Proteose peptone n° 3 Bacto | 10 g |
| Extrait de levure bacto | 10 g |
| Extrait de levure bacto | 5 g |
| Dextrose bacto | 20 g |
| Tween 80 | 1 g |
| Citrate d'ammonium | 2 g |
| Acétate de sodium | 5 g |
| Sulfate de magnésium | 0,1 g |
| Sulfate de manganèse | 0,005 g |
| Phosphate de dipotassium | 2 g |

resuspension de 55 g de poudre dans 1 litre d'eau ;
pH final 6,5 + 0,2 à 25°C.

Une culture préliminaire de la souche bactérienne à tester est indispensable avant de réaliser le test. Les bactéries sont cultivées dans du milieu MRS liquide à 37°C en condition microaérophile. Elles sont ensuite séparées du surnageant par centrifugation et resuspendues dans une solution saline de tampon phosphate (PBS), (pH 7,3). La solution de bactéries est ajustée à une densité optique (DO) de 5 (DO de 0,5 à 600 nm = 3.10⁸ bactéries par ml) puis diluée au ½ vingt-quatre fois. Le surnageant pur est dilué au ½ douze fois.

Ce test consiste à adsorber le récepteur intestinal dans les micropuits de plaques ELISA pendant une nuit à 4°C dans une solution phosphate saline. Les espaces entre les récepteurs sont comblés par un agent saturant (BSA à 1 %) pendant 30 minutes à température ambiante. Après trois étapes de lavage, 50 µl de chacune des solutions de la gamme des souches bactériennes (ou de surnageant de culture) sont déposés successivement dans 24 ou 12 puits respectivement. Après 12 heures d'incubation à 4°C ou 2 heures d'incubation à 37°C, 50 µl de l'anticorps monoclonal 7E8aB4 de concentration constante à 0,24 µg/ml sont ajoutés à tous les puits. La concentration d'anticorps a été préalablement ajustée pour obtenir un signal satisfaisant de l'ordre de DO=1,2 en ELISA. Une série de trois lavages est à nouveau réalisée, et le reste du test consiste à détecter la présence de 7E8aB4 dans les puits en utilisant des anticorps conjugués à la peroxydase, qui sont susceptibles de reconnaître 7E8aB4. Un substrat de l'enzyme (O-phénylène-diamine) est distribué dans tous les puits de la plaque ELISA. Ainsi, si le conjugué anticorps-récepteur est présent dans un puits, l'enzyme dégrade le substrat en développant une réaction colorée localisée dont l'intensité est directement dépendante de la quantité d'anticorps présente. Les valeurs de densité optique de chaque puits sont précisément déterminées par un lecteur de plaques ELISA.

### b) Résultats :

Les valeurs de densité optique sont comparées à celles des témoins positifs (en présence d'anticorps 7E8aB4 uniquement). Une concentration constante en anticorps monoclonaux donne un signal coloré constant, et donc une valeur DO proche de 1,2. Si les bactéries testées sont capables d'empêcher la fixation de l'anticorps, les valeurs de la DO doivent chuter significativement en corrélation avec le nombre de bactéries présentes. Les courbes obtenues sont différentes suivant les souches testées. Le profil des courbes est analysé et comparé à celui d'un témoin négatif représenté par une souche de référence connue pour son absence d'adhésion (souche n° 3A de la figure 1 annexée). Ce témoin négatif permet également de différencier une inhibition spécifique d'une inhibition non spécifique de la fixation de l'anticorps. Les inhibitions non spécifiques sont causées par l'encombrement des corps bactériens pour des concentrations élevées en microorganismes. L'objectif est de rechercher les bactéries capables d'exercer l'inhibition la plus marquée (DO la plus faible) pour la dilution la plus élevée possible en microorganismes en se référant au profil des témoins négatifs et positifs.

Lorsque le profil obtenu avec une souche est presque superposable avec celui du témoin négatif, et que de plus la valeur de DO augmente très rapidement dès les premières dilutions pour atteindre le palier de la DO maximale fixé à la valeur de 1,2, on peut conclure que cette souche n'empêche pas la fixation de l'anticorps sur les récepteurs (souche n° 36S de la figure 1 annexée). En revanche, par rapport au témoin, certaines souches exercent une activité inhibitrice prolongée pour de faibles concentrations en bactéries (exemple des souches n° D1, 27S et 30S sur la figure 1 annexée). Les courbes obtenues sont aisément différenciables de celles des souches témoin non adhérentes, car elles sont décalées vers la droite sur la figure annexée (sens des concentrations faibles en bactéries). Plus les courbes sont décalées vers la droite, plus les souches bactériennes sont intéressantes et efficaces pour être en compétition avec les souches ETEC sur le site d'adhésion de fimbriae de K88ac.

### • Souches n° 36S et LB14 :

La souche 36S a été isolée du colon d'un porcelet âgé de 6 semaines et a été identifiée comme étant une bactérie lactique faisant partie du genre *Lactobacillus* et du groupe I des lactobacilles homofermentaires stricts Lactobacille appartenant au premier complexe, espèce de *Lactobacillus delbrueckii,* sous-espèce à déterminer par analyse fine en PCR.

La souche LB14 a été fraîchement isolée du duodénum de porcelets âgés de 6 semaines. Il s'agit d'une bactérie lactique faisant partie du genre Lactobacillus et du groupe Il des lactobacilles hétérofermentaires facultatifs, Lactobacille appartenant au deuxième complexe, espèce de *Lactobacillus casei,* sous-espèce à déterminer par analyse fine.

Pour les souches 36S et LB14, dès que la dilution en bactéries augmente, les valeurs de la densité optique augmentent elles aussi très rapidement en suivant le profil du témoin négatif et en atteignant la densité optimale pour des dilutions fiables de bactéries non pathogènes. L'inhibition observée est essentiellement causée par un nombre très élevé de bactéries dans les puits des plaques ELISA empêchant de ce fait l'accès de l'anticorps vers son récepteur fixé au fond des puits. Dans le cadre d'un tel encombrement cellulaire non spécifique, une inhibition de 50 % (DO de 0,4) se rapproche d'une charge de 12.10⁶ bactéries/puits. On peut considérer que les souches présentant des résultats similaires ne sont pas intéressantes pour entreprendre un test de challenge *in vivo.*

### • Souches 30S (L. fermentum) et 27S (L. fermentum):

Les souches 27S et 30S ont été isolées du petit intestin de porcs âgés de 6 à 16 semaines et ont été identifiées comme étant deux bactéries lactiques faisant partie du genre *Lactobacillus* et du groupe III des lactobacilles hétérofermentaires stricts, espèce de *Lactobacillus fermentum.*

Dans ces exemples, on constate une différence très nette avec le profil des souches décrit précédemment. En effet, malgré plusieurs dilutions aux 1/2 des bactéries non pathogènes, l'inhibition persiste car les valeurs de la densité optique restent stables et proches de la ligne de base. Les premières phases de l'inhibition sont causées par un encombrement bactérien non spécifique mais se prolongent ensuite par une inhibition plus spécifique sur le site récepteur de notre outil immunologique. Il faut une dilution plus élevée en bactéries non pathogènes que celle du témoin négatif pour observer la levée progressive de l'inhibition. Pour 50 % d'inhibition (DO de 0,02), la charge en bactéries dans le puits est de l'ordre de 60.10⁴ soit 20 fois moins concentrée que la charge en bactéries du témoin négatif et des souches 36S et LB14. Ces souches sont sélectionnées pour poursuivre des tests expérimentaux *in vivo.*

### • Souche D1 :

La souche D1 est une bactérie lactique faisant partie du genre *Lactobacillus* et du groupe I des lactobacilles homofermentaires stricts, espèce de *Lactobacillus salivarius.*

Malgré le fait que l'inhibition soit levée plus tôt que celle des souches 27S et 30S, elle est cependant légèrement plus durable. En conséquence, pour 50 % d'inhibition (DO de 0,03), la charge en bactéries non pathogènes dans le puits est de l'ordre de 90.10⁴ soit 1.5 fois plus concentrée que celle des souches 27S et 30S mais reste tout de même 13 fois moins concentrée que la charge en bactéries du témoin négatif et des souches 36S et LB 14. Ce profil d'inhibition intermédiaire se rapproche donc de celui des souches sélectionnées. La souche D1 est donc un bon candidat pour les tests de compétition in *vivo.*

La présente invention permet donc de sélectionner des souches de bactéries non pathogènes susceptibles de s'opposer spécifiquement à la fixation des bactéries entérotoxinogènes donc à l'expression de leur pouvoir pathogène. L'administration préventive des souches non pathogènes sélectionnées permet d'éviter l'apparition des troubles provoqués par une infection par des bactéries entérotoxinogènes, tels que des diarrhées. Leur administration à titre curatif permet d'atténuer voire de supprimer ces mêmes troubles.

### V - La confirmation in vitro des capacités inhibitrices de souches sélectionnées

### a) matériels et méthodes

Afin d'évaluer les capacités inhibitrices des surnageants de culture et de bactéries lavées vis-à-vis de l'anticorps, un test de contrôle a été entrepris en remplaçant l'anticorps par des fimbriae de K88ac purifiées. Dans ce cas, on utilise un sérum polyclonal anti-K88ac et des anticorps conjugués anti-sérum pour la détection de la présence de fimbriae.

Dans ce test, le récepteur intestinal est déposé dans les micropuits de plaques ELISA puis laissé pendant une nuit à 4°C dans une solution phosphate saline. Les espaces entre les récepteurs sont comblés par un agent saturant (serumalbumine bovine BSA à 1 %) pendant 30 minutes à température ambiante. Après trois étapes de lavage, 50 µl de chacune des solutions de la gamme des souches préalablement sélectionnées (ou de surnageant de culture) sont déposés successivement dans 24 ou 12 puits respectivement. Après douze heures d'incubation à 4°C ou deux heures d'incubation à 37°C, 50 µg de préparation de fimbriae K88ac de concentration constante à 12,5 µg/ml sont ajoutés à tous les puits. La concentration de fimbriae a été préalablement ajustée pour obtenir un signal satisfaisant de l'ordre de DO=1,2 en ELISA. Une série de trois lavages est à nouveau réalisée et le reste du test consiste à détecter la présence des fimbriae K88ac dans les puits en utilisant des anticorps polyclonaux obtenus à partir d'un anti-sérum de lapin. Les anticorps de lapin ayant reconnu les fimbriae sont eux-mêmes détectés après lavage par des anticorps conjugués à la biotine. Un complexe enzymatique (streptavidine/peroxydase de raifort) capable de reconnaître la biotine est ensuite ajouté. Enfin, un substrat de l'enzyme (O-phénylène-diamine) est distribué dans tous les puits des plaques ELISA pour le développement de la réaction colorée. Ces cascades de reconnaissance sont nécessaires car les fimbriae K88ac sont très hydrophobes et l'utilisation d'une concentration trop élevée en fimbriae peut entraîner l'apparition de fixations non spécifiques des lectines bactériennes. Une molécule de complexe streptavidine porte plusieurs enzymes peroxydases. Ainsi, l'utilisation de ces complexes enzymatiques permet de travailler avec des concentrations faibles en fimbriae tout en amplifiant le signal de coloration.

### b) résultats

Les valeurs de densité optiques ont été comparées à celles des témoins ne comportant que les fimbriae K88ac sur le récepteur (figure 2). Une concentration constante en fimbriae a donné un signal coloré constant et donc une valeur de DO proche de 1,2. D'après les résultats, la fixation des fimbriae est plus difficile à inhiber que celle de l'anticorps 7E8aB4. Il faut en effet plus de bactéries par puits pour observer une inhibition des fimbriae comparable à celle du test utilisant l'anticorps monoclonal. Cette différence peut être expliquée par une différence entre le nombre de sites de fixation de l'anticorps utilisé et de la fimbriae. Une molécule d'IgM porte dix sites de reconnaissance alors qu'une fimbriae en porte certainement beaucoup plus. Cependant, malgré cette différence, le classement des souches sélectionnées d'après leur activité inhibitrice ne change pas.

### VI - Validation in vivo de l'efficacité des souches sélectionnées

Le processus décrit précédemment aboutit à la sélection de souches bactériennes non pathogènes se liant spécifiquement sur les récepteurs des fimbriae de *E. Coli* K88ac de la muqueuse intestinale de porcelets. Il est ensuite nécessaire de valider *in vivo* cette fixation, grâce à laquelle la souche bactérienne sélectionnée peut se multiplier dans l'intestin grêle et assurer une prévention efficace contre la fixation de *E. Coli* K88ac.

A cet effet, des porcelets sont mis à l'écart dès la naissance et placés dans des isolateurs avant ingestion de colostrum. Ils reçoivent des aliments liquides classiques. De ce fait, ces porcelets ne sont pas dépourvus de flore microbienne, mais ne bénéficient pas de la protection immunitaire du colostrum.

Ces porcelets reçoivent dans leur alimentation une souche bactérienne non pathogène telle que sélectionnée précédemment pendant trois jours. On leur administre ensuite une dose de 5 x 10⁸ CFU d'une souche toxicogénique de *E. coli* K88ac. Les porcelets sont euthanasiés 24 heures après cette administration, et on mesure dans les différents segments de l'intestin grêle comme du caecum la concentration en germes totaux, en *E. coli* et en la souche bactérienne non pathogène sélectionnée. Par ailleurs, on détermine la sensibilité génétique de ces porcelets vis à vis de *E. coli* K88ac.

Si les porcelets sensibles, donc porteurs de sites d'adhésion, ont une concentration significativement plus élevée de la souche bactérienne non pathogène sélectionnée que les porcelets résistants, ceci signifie que cette souche a pu se fixer sur les sites d'adhésion et se multiplier. Cette souche bactérienne sera donc susceptible de modifier la flore microbienne de l'intestin grêle dans un sens défavorable à *E. coli* K88ac.

## Revendications

1. Procédé de sélection de souches bactériennes non pathogènes capables de se fixer sur les sites récepteurs d'adhésion tissulaire de souches bactériennes pathogènes, comprenant les étapes consistant à :
a) mettre en contact *in vitro* lesdits récepteurs avec une souche bactérienne à tester ;
b) ajouter un anticorps dirigé contre lesdits récepteurs, ledit anticorps étant éventuellement marqué de manière détectable ;
c) dans le cas où ledit anticorps n'est pas lui-même marqué de manière détectable, ajouter un agent de détection de l'anticorps ;
d) détecter la présence du complexe formé par ledit anticorps et lesdits récepteurs.

2. Procédé selon la revendication 1 dans lequel ledit anticorps dirigé contre le récepteur spécifique de souches bactériennes pathogènes appelé premier anticorps, n'est pas marqué de manière détectable mais peut être détecté par ajout d'un agent de détection selon l'étape c) de la revendication 1, ledit agent de détection étant un deuxième anticorps marqué de manière détectable et dirigé contre ledit premier anticorps.

3. Procédé selon l'une quelconque des revendications 1 ou 2, dans lequel lesdits récepteurs sont des récepteurs spécifiques de la paroi intestinale sur lesquels se fixent les souches entérotoxinogènes d'*Escherischia coli.*

4. Composition thérapeutique comprenant une souche bactérienne sélectionnée par le procédé selon l'une quelconque des revendications 1 à 3.

5. Utilisation des souches bactériennes non pathogènes sélectionnées par le procédé selon l'une quelconque des revendications 1 à 3 pour la fabrication d'une composition thérapeutique destinée à prévenir ou traiter les troubles pathologiques associés à une infection d'un hôte animal par des souches bactériennes pathogènes se fixant sur lesdits récepteurs médiant l'adhésion tissulaire.

6. Utilisation selon la revendication 5 pour prévenir ou traiter les troubles pathologiques associés à une infection par des souches entérotoxinogènes d*'Escherischia coli.*

7. Utilisation selon l'une quelconque des revendications 5 ou 6. dans laquelle l'hôte animal est le porc.
